# EUROPEAN PATENT SPECIFICATION

(11) **EP 1 089 767 B1**
(45) Date of publication and mention of the grant of the patent: **23.06.2004**
(21) Application number: 99929951.4
(22) Date of filing: 23.06.1999
(51) Int. Cl.: A61L 2/08

(54) **METHOD OF INACTIVATING VIRUSES**
VERFAHREN ZUR INAKTIVIERUNG VON VIREN
METHODE D'INACTIVATION DE VIRUS

(30) Priority: 23.06.1998 NL 1009472
(43) Date of publication of application: 11.04.2001
(73) Proprietor: PhotoBioChem N.V., 2333 AL Leiden (NL); Stichting Sanquin, 1066 CX Amsterdam (NL)
(72) Inventor: VAN STEVENINCK, Johnny, (NL); DUBBELMAN, Thomas, Marinus, Albert, Remko, NL-2406 JJ Alphen a/d Rijn (NL); LAGERBERG, Johannes, Wilhelminus, Maria, NL-2317 DV Leiden (NL); BRAND, Agatha, NL-1016 AE Amsterdam (NL); VERHOEVEN, Arthur, Johan, NL-1078 MA Amsterdam (NL)
(74) Representative: Kupecz, A., Drs. c.s.
(86) International application number: PCT/NL1999/000387
(87) International publication number: WO 1999/066962

(56) References cited:
- WO-A-91/16060
- WO-A-95/20961
- WO-A-97/15661
- BEN-HUR E ET AL: "Virus inactivation in red cell concentrates by photosensitization with phthalocyanines: protection of red cells but not of vesicular stomatitis virus with a water-soluble analogue of vitamin E." TRANSFUSION, (1995 MAY) 35 (5) 401-6. JOURNAL CODE: WDN. ISSN: 0041-1132., XP002095432 United States

## Description

The present invention relates to a method of inactivating viruses in an erythrocytes-containing liquid with the use of light, wherein a sensitizer and an erythrocyte-binding agent are added to the liquid which erythrocyte-binding agent protects against the influence of singlet oxygen and/or radicals.

In the Dutch patent application 1006219 (not pre-published) a method is described wherein a sensitizer is added to an erythrocytes-containing liquid such as, for example, diluted blood, after which the liquid is irradiated with light, thereby exciting the sensitizer. Interaction between the excited sensitizer and oxygen present results in reactive oxygen species causing a virus present to become inactivated. Other interactions of the excited sensitizer may result in the occurrence of radicals and other reactive species, which may also cause a virus present to become inactivated.

A disadvantage of applying such a known method of inactivating viruses is that apart from the virus, the erythrocytes themselves are damaged by reactive oxygen species. This limits the extent to which the viruses can be inactivated.

A method according to the preamble is known from Ben-Hur et al.: Transfusion, (1995 May) 35 (5) 401-6. Ben-Hur et al. discloses virus inactivation in red cell concentrates by photosensitization with phthalocyanines: protection of red cells but not of vesicular stomatitis virus (VSV) with a water-soluble analogue of vitamin E.

The erythrocyte-binding agent according to Ben-Hur, a water-soluble analogue of vitamin E, binds to a membrane protein, resulting in a non-selective protection. The erythrocyte-binding agent according to Ben-Hur reduces potassium leakage by about 50% (p404, left column), which signifies that still a considerable cell damage has been incurred.

It is the object of the present invention to provide a better protection. According to the present invention this object is realized by a method of inactivating viruses in an erythrocytes-containing liquid with the use of light, wherein a sensitizer and an erythrocyte-binding agent are added to the liquid, which erythrocyte-binding agent protects against the influence of singlet oxygen and/or radicals, characterized in that the protecting agent is selected from the group consisting of i) a ligand for a transport protein, and ii) an agent bound to a carrier capable of binding to the erythrocyte's surface.

As can be seen from the Examples below, the addition of the agent according to the invention provides the erythrocyte with a greater degree of protection against photodynamic damage than the virus particle.

According to a preferred embodiment, the agent is bound to a carrier binding to the erythrocyte's surface, preferably covalently. This means that more agent molecules are available. Moreover, the specificity may be chosen freely. Preferably, said carrier is an antibody against a surface antigen of the erythrocyte, such as IgM.

IgM binds weakly so that it can later be easily removed again.

In accordance with another feature of the invention the transport protein is preferably an anion transport protein.

Such transport proteins occur in relatively large numbers on the cell surface, so that they conveniently provide for the possibility of protecting the cell surface.

In accordance with another feature of the invention the transport protein is a Band 3-protein forming part of a complex that, among other things, provides for HCO₃⁻ transport that is important for erythrocytes. The "concentration" of Band 3-proteins at the cell surface is high (~10⁶ copies per cell). Apart from that, no/virtually no Band 3-relaxed structures occur on the surface of membrane virus particles, so that Band 3-specific ligands will have a much lower affinity for the surface of the virus particle. Moreover, Band 3 has been studied extensively and there are many ligands known that bind to Band 3.

According to one embodiment, the ligand binds to a transport site of the transport protein, the ligand preferably being pyridoxal phosphate (PDP) or a derivative thereof. PDP is a vitamin, so that its retention in treated erythrocyte suspension in relatively small amounts will pose no problems. It should be noted that when PDP (which is itself also a sensitizer) is used, the wave-length of the light used must be chosen such that only the compound added and intended as sensitizer will be excited, and not PDP.

According to another embodiment the ligand binds to the protein's channel domain, the ligand preferably being dipyridamol (DIP) or a derivative thereof. DIP is a registered drug, the use of which is known to cause limited side effects.

According to yet another embodiment the ligand binds to the protein's conformation site. Such a ligand may, for example, be a translocation inhibitor such as, for example, nifluminic acid.

The invention will now be elucidated with reference to the exemplary embodiments.

### Example 1

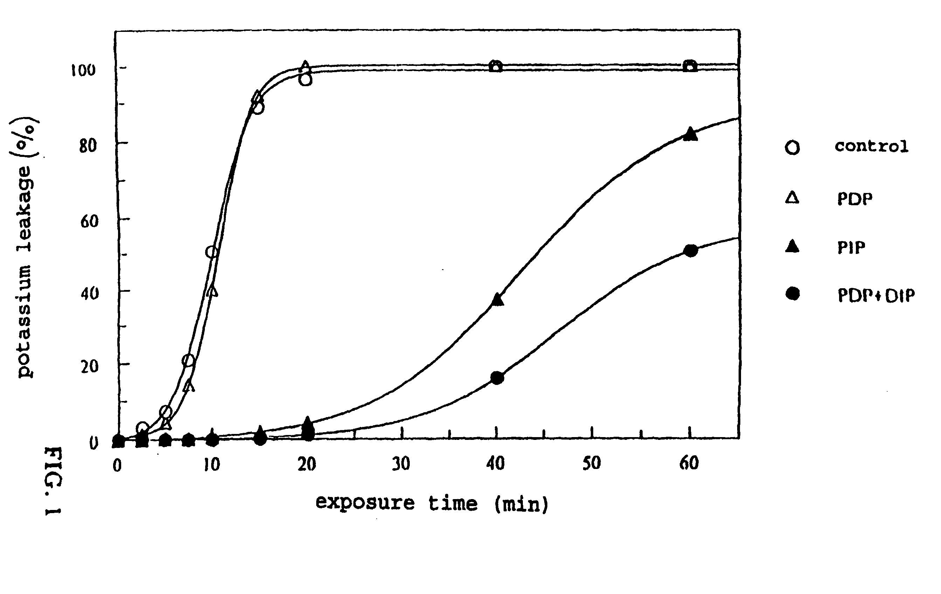

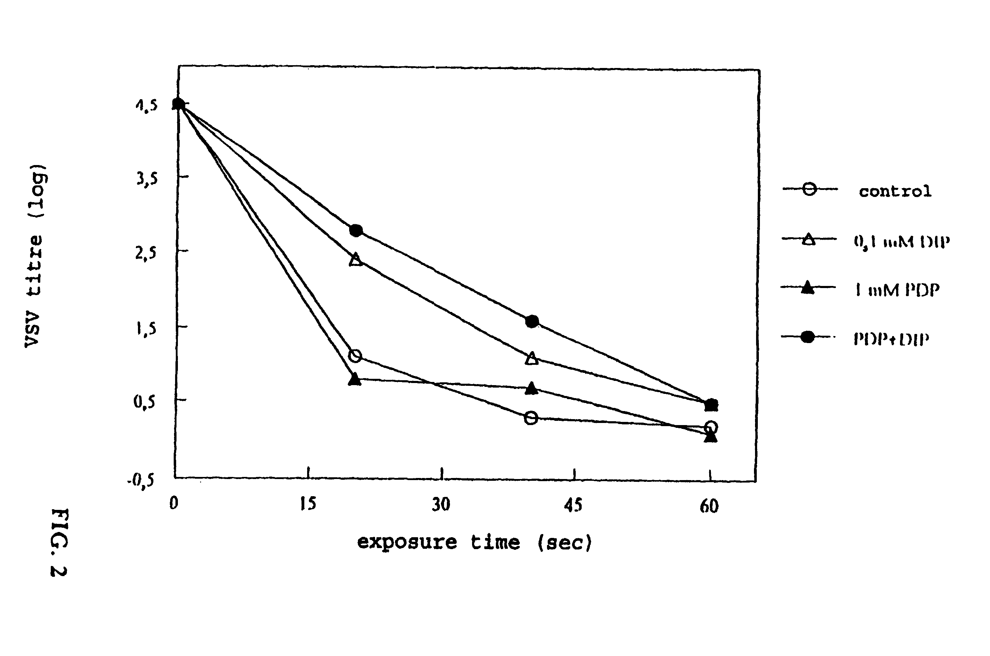

Shortly after blood samples were taken from healthy donors, the blood was centrifuged, and the erythrocytes were washed three times with NaCl-HEPES buffer (150 mM NaCl-10 mM HEPES (ICN Biochemicals, Cleveland Ohio, USA) pH 7.6). Dilution in this buffer provided a 2% erythrocyte suspension. 10⁵ Vesicular Stomatitis virus (VSV) particles per ml were added (San Juan strain, obtained from the Department of Virology, LUMC, Leiden). Further, dimethyl-methylene blue (Aldrich, the Netherlands) was added as sensitizer, in a final concentration of 1 µM. The erythrocyte suspension obtained was divided into four portions (A, B, C and D). Nothing was added to portion A; to portion B 1 mM pyridoxal phosphate PDP was added; to C 0.1 mM dipyridamol DIP; and to D 1 mM pyridoxal phosphate plus 0.1 mM dipyridamol. Subsequently the portions A, B, C and D were irradiated with light from a 500 W halogen lamp, which light was passed through a water filter (to eliminate heat effects) and then through a low-pass filter having a cut-off value of 590 nm. The final light intensity was 15 mW/cm². The following factors were established:
- potassium leakageage (K leakage) from erythrocytes after different exposure times (Fig. 1);
- virus inactivation after different exposure times (Fig. 2).

The combination of pyridoxal phosphate/dipyridamol was shown to provide the virus with little protection against photodynamic inactivation, while (particularly after short exposure times) the erythrocyte was well protected against (photodynamically caused) cell wall damage, as appears from the small K-leakage. A large K-leakage signifies that considerable cell damage has been incurred.

### Example 2

A control experiment was carried out to show that light exposure of the erythrocyte suspension without the sensitizer does not cause any photodynamic damage to the erythrocyte and/or the virus.

The experiment of Example 1 was repeated, this time in the presence of dipyridamol and/or pyridoxal phosphate, but in the absence of the sensitizer dimethyl-methylene blue. Light exposure of the erythrocyte suspension did indeed have no effect at all with regard to the virus inactivation (results not shown).

### Example 3

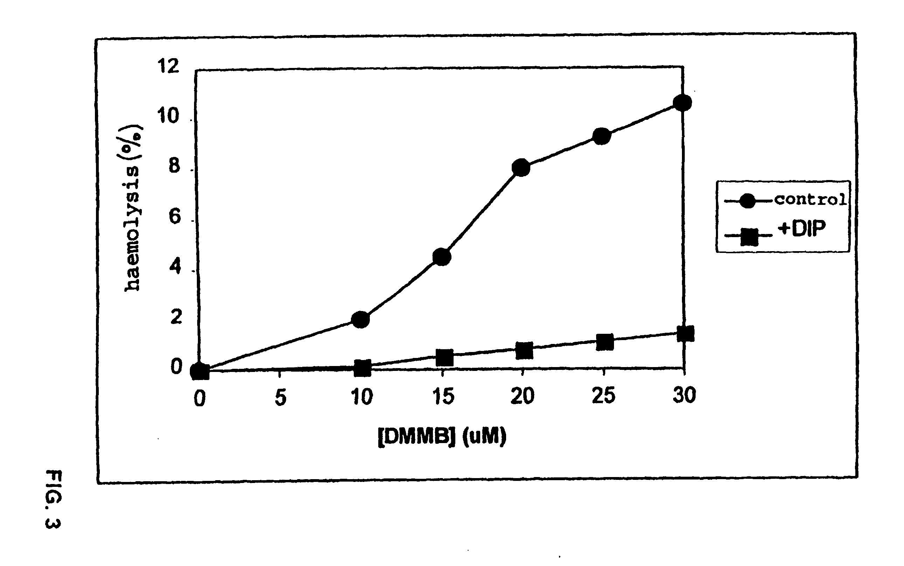

The possible interference of erythrocyte ligands with the efficiency of virus inactivation was also studied in more concentrated erythrocyte suspensions (30% haematocrit). For the photodynamic treatment dimethyl-methylene blue (DMMB), red light (as in Example 1) and dipyridamol as erythrocyte ligand and scavenger. In these experiments the minimal DMMB concentration was determined, that was necessary to effectuate at an exposure time of 2 minutes, a more than ³log virus titre reduction of Vesicular Stomatitis (VSV, strain Indiana, obtained from the Department of Biotechnology CLB, Leiden, the Netherlands), PseudoRabies virus (PSR, strain Bartha K61, obtained from Duphar, Weesp) and Humane Immunodeficiency Virus Type 1 (HIV-1, strain HTLV-IIIb, obtained from the National Cancer Institute, Bethesda, United States of America). To this end 10⁵ virus particles per ml (for VSV and PSR) or 10⁶ virus particles per ml (for HIV-1) were incubated for 5 minutes with humane erythrocytes (30% Ht, suspended in 150 mM NaCl, 50 mM glucose, 30 mM mannitol and 1.2 mM adenine), after which dipyridamol was added to half the incubations (final concentration 0.1 mM). After a further 10 minutes incubation at room temperature, increasing amounts (0-16 µM) of DMMB were added to the incubations which were then incubated in the dark for 6 minutes at room temperature before exposing the suspensions for 2 minutes to light. The addition of dipyridamol (0.1 mM) did not, or only barely appear to affect the effective concentration of sensitizer (Table I, below) and consequently did not appear to decrease the efficiency of virus inactivation. The addition of dipyridamol did, however, result in effective protection of the erythrocytes, which was expressed, among other things, by a very strong reduction of the haemolysis measured after the erythrocytes had been stored for 12 days at 4°C. Fig. 3 shows the results of haemolysis measurements 12 days after the photodynamic treatment (2 minutes light exposure and increasing DMMB concentrations).

**TABLE 1**

| virus | effective [DMMB] (µM) | |
|---|---|---|
| | control | + DIP |
| VSV | 4 | 5 |
| PSR | 4 | 4 |
| HIV-1 | 8 | 8 |

## Claims

1. A method of inactivating viruses in an erythrocytes-containing liquid with the use of light, wherein a sensitizer and an erythrocyte-binding agent are added to the liquid, which erythrocyte-binding agent protects against the influence of singlet oxygen and/or radicals, **characterized in that** the protecting agent is selected from the group consisting of i) a ligand for a transport protein, and ii) an agent bound to a carrier capable of binding to the erythrocyte's surface.

2. A method according to claim 1, **characterized in that** the transport protein is an anion transport protein.

3. A method according to claim 1 or 2, **characterized in that** the transport protein is a Band 3-protein.

4. A method according to one of the preceding claims, **characterized in that** the ligand binds to a transport site of the transport protein.

5. A method according to claim 4, **characterized in that** the ligand is pyridoxal phosphate (PDP) or a derivative thereof.

6. A method according to one of the preceding claims, **characterized in that** the ligand binds to the protein's channel domain.

7. A method according to claim 6, **characterized in that** the ligand is dipyridamol (DIP) or a derivative thereof.

8. A method according to one of the preceding claims, **characterized in that** the ligand binds to the protein's conformation site.

9. A method according to claim 8, **characterized in that** the ligand is nifluminic acid.

10. A method according to claim 1, **characterized in that** the carrier is an antibody against a surface antigen of the erythrocyte.

11. A method according to claim 10, **characterized in that** the antibody is IgM.

## Patentansprüche

1. Verfahren zur Deaktivierung von Viren in einer Erythrozyten enthaltenden Lösung unter Einsatz von Licht, wobei ein Sensibilisator und ein Erythrozyten bindendes Mittel zu der Flüssigkeit gegeben werden, wobei das Erythrozyten bindende Mittel als Schutz vor dem Einfluss von Singulett-Sauerstoff und/oder Radikalen dient, **dadurch gekennzeichnet, dass** das schützende Mittel ausgewählt ist aus der Gruppe, bestehend aus i) einem Liganden für ein Transport-Protein und ii) einem Mittel, das an einen Träger gebunden ist, der in der Lage ist, mit der Oberfläche des Erythrozyten eine Bindung einzugehen.

2. Verfahren gemäß Anspruch 1, **dadurch gekennzeichnet, dass** das Transport-Protein ein anionisches Transport-Protein ist.

3. Verfahren gemäß Anspruch 1 oder 2, **dadurch gekennzeichnet, dass** das Transport-Protein ein Band 3-Protein ist.

4. Verfahren gemäß einem der vorangegangenen Ansprüche, **dadurch gekennzeichnet, dass** der Ligand an der Transportseite des Transport-Proteins anbindet.

5. Verfahren gemäß Anspruch 4, **dadurch gekennzeichnet, dass** der Ligand Pyridoxalphosphat (PDP) oder ein Derivat davon ist.

6. Verfahren gemäß einem der vorangegangenen Ansprüche, **dadurch gekennzeichnet, dass** der Ligand an der Kanal-Domäne des Proteins anbindet.

7. Verfahren gemäß Anspruch 6, **dadurch gekennzeichnet, dass** der Ligand Dipyridamol (DIP) oder ein Derivat davon ist.

8. Verfahren gemäß einem der vorangegangenen Ansprüche, **dadurch gekennzeichnet, dass** der Ligand an der Kontormationsseite des Proteins anbindet.

9. Verfahren gemäß Anspruch 8, **dadurch gekennzeichnet, dass** der Ligand Nifluminsäure ist.

10. Verfahren gemäß Anspruch 1, **dadurch gekennzeichnet, dass** der Träger ein Antikörper zu einem Oberflächen-Antigen des Erythrozyten ist.

11. Verfahren gemäß Anspruch 10, **dadurch gekennzeichnet, dass** der Antikörper IgM ist.

## Revendications

1. Procédé d'inactivation de virus dans un liquide contenant des érythrocytes par l'utilisation de lumière, procédé dans lequel un agent sensibilisant et un agent se liant à l'érythrocyte sont ajoutés au liquide, ledit agent se liant aux érythrocytes protégeant contre l'influence d'un oxygène singlet et/ou des radicaux, **caractérisé par le fait que** l'agent protecteur est choisi dans le groupe constitué de i) un ligand pour une protéine de transport et ii) un agent fixé à un transporteur capable de se fixer à la surface de l'érythrocyte.

2. Procédé selon la revendication 1, **caractérisé par le fait que** la protéine de transport est une protéine de transport anionique.

3. Procédé selon la revendication 1 ou 2, **caractérisé par le fait que** la protéine de transport est une protéine-Bande 3.

4. Procédé selon l'une quelconque des revendications précédentes, **caractérisée par le fait que** le ligand se fixe à un site de transport de la protéine de transport.

5. Procédé selon la revendication 4, **caractérisé par le fait que** le ligand est le phosphate de pyridoxal (PDP) ou un dérivé de celui-ci.

6. Procédé selon l'une quelconque des revendications précédentes, **caractérisé par le fait que** le ligand se fixe au domaine tunnel de la protéine.

7. Procédé selon la revendication 6, **caractérisé par le fait que** le ligand est le dipyridamol (DIP) ou un dérivé de celui-ci.

8. Procédé selon l'une quelconque des revendications précédentes, **caractérisé par le fait que** le ligand se fixe au site de conformation de la protéine.

9. Procédé selon la revendication 8, **caractérisé par le fait que** le ligand est l'acide nifluminique.

10. Procédé selon la revendication 1, **caractérisé par le fait que** le transporteur est un anticorps dirigé contre un antigène de surface de l'érythrocyte.

11. Procédé selon la revendication 10, **caractérisé par le fait que** l'anticorps est un IgM.
